# EUROPEAN PATENT APPLICATION

(11) **EP 4 602 912 A1**
(43) Date of publication of application: **20.08.2025**
(21) Application number: 23876986.3
(22) Date of filing: 16.08.2023
(51) Int. Cl.: A01K 1/01, A01K 23/00

(54) **ANIMAL TOILET**

(30) Priority: 14.10.2022 JP 2022165114
(71) Applicant: Daiki Co., Ltd., Tokyo 102-0072 (JP)
(72) Inventor: YOSHINAGA Junji, Tokyo 107-0052 (JP)
(74) Representative: Müller-Boré & Partner Patentanwälte PartG mbB
(86) International application number: PCT/JP2023/029589
(87) International publication number: WO 2024/079978

(57) **Abstract**

Provided is an animal toilet in which fresh urine can be easily collected. An animal toilet (1) includes a body portion (10). The body portion (10) has a bottom face part (10a) and a side face part (10b). A grain (20) for treating urine of an animal is laid in the body portion (10). The side face part (10b) is provided with an opening (12). The opening (12) is for inserting a urine collection tool (30) used for collecting the urine above the grain (20).

## Description

### Technical Field

The present invention relates to an animal toilet.

### Background Art

A conventional animal toilet is disclosed, for example, in Patent Document 1. The animal toilet disclosed in Patent Document 1 includes a box-shaped body portion (toilet body) that has a bottom face part and a side face part. An excrement treatment material composed of a plurality of grains is laid in the body portion.

### Citation List

### Patent Document

Patent Document 1: JP 2018-88881 A

### Summary of Invention

### Technical Problem

Incidentally, a urine test is sometimes conducted in order to check a health condition of an animal. To conduct a urine test accurately, it is required to collect fresh urine (urine that has not touched the body portion or the grains). In the animal toilet described above, it is possible to collect fresh urine by inserting a urine collection tool under the animal's rear from above the body portion when the animal urinates. However, if there is insufficient space between the animal's body and the side face part of the body portion, it is difficult to insert the urine collection tool under the animal's rear. For that reason, users (such as animal owners) have been struggling to collect fresh urine in the conventional animal toilet.

The present invention has been made in view of the above-described problem, and it is an object thereof to provide an animal toilet in which fresh urine can be easily collected.

### Solution to Problem

An animal toilet according to the present invention includes: a body portion in which a grain for treating urine of an animal is laid, the body portion having a bottom face part and a side face part, wherein the side face part is provided with an opening for inserting a urine collection tool used for collecting the urine above the grain.

In this animal toilet, the side face part of the body portion is provided with the opening for inserting the urine collection tool above the grain. For this reason, even in the case where the space between the animal's body and the side face part is small, the urine collection tool can be easily inserted under the animal's rear (between the rear and the grain) through the opening of the side face part.

### Advantageous Effects of Invention

According to the present invention, it is possible to implement an animal toilet in which fresh urine can be easily collected.

### Brief Description of Drawings

FIG. 1 is an end view showing a first embodiment of an animal toilet according to the present invention.
FIG. 2 is a plane view showing a body portion 10.
FIG. 3 is a front view showing the body portion 10.
FIG. 4 is a front view showing an opening 12.
FIG. 5 is a schematic view showing a grain 20.
FIG. 6 is a perspective view showing a urine collection tool 30.
FIG. 7 is a schematic view showing a state in which the urine collection tool 30 is inserted into the opening 12.
FIG. 8 is an end view showing a second embodiment of an animal toilet according to the present invention.
FIG. 9 is a plane view showing a body portion 40.
FIG. 10 is a front view showing the body portion 40.
FIG. 11 is an end view showing the body portion 40.
FIG. 12 is a plane view showing a partition portion 50.
FIG. 13 is a perspective view showing a drawer portion 80.
FIG. 14 is a front view showing the opening 12 according to a modified example.
FIG. 15 is a front view showing the opening 12 according to another modified example.

### Description of Embodiments

Hereinafter, embodiments of the present invention will be described in detail with reference to the drawings. In the description of the drawings, the same elements are given the same reference numerals, and a redundant description will be omitted.

### (First Embodiment)

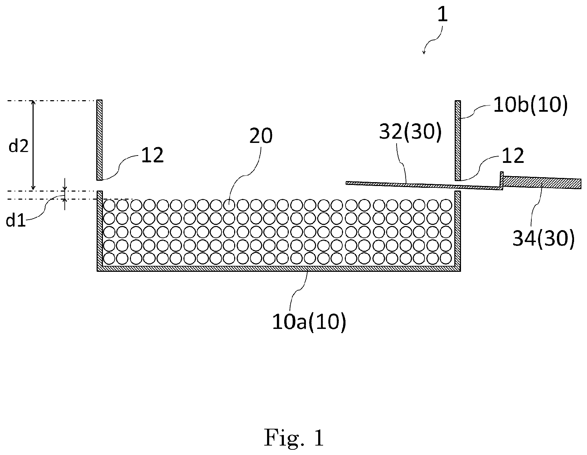

FIG. 1 is an end view showing a first embodiment of an animal toilet according to the present invention. An animal toilet 1 includes a body portion 10, a grain 20, and a urine collection tool 30. The animal toilet 1 is, for example, a cat toilet. The body portion 10 is composed of one box-shaped container, and has a bottom face part 10a and a side face part 10b. The body portion 10 receives excreted urine. A plurality of the grains 20 for treating animal urine are laid in the body portion 10. The outer shape of the body portion 10 is an approximately rectangular parallelepiped shape. As a material of the body portion 10, for example, plastic such as polypropylene or polyethylene can be used.

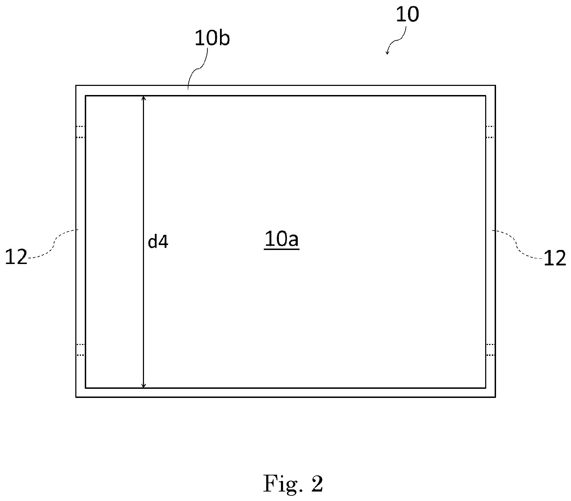

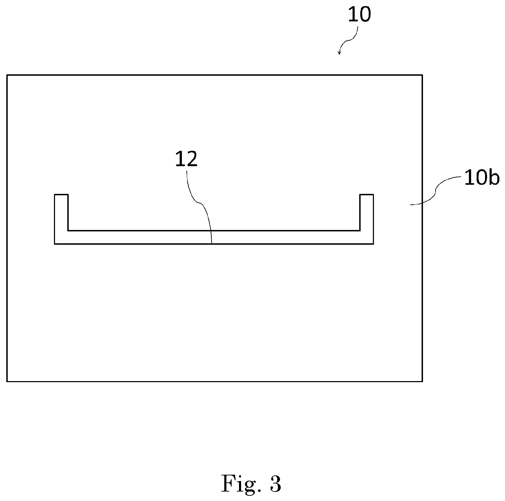

FIG. 2 and FIG. 3 are, respectively, a plane view and a front view showing the body portion 10. The bottom face part 10a has an approximately rectangular shape in a plane view. As used herein, "approximately rectangular shape" means that the shape includes not only a rectangle but also a shape similar to a rectangle such as a round corner rectangle. The side face part 10b is composed of four parts that are connected, respectively, to the four sides of the bottom face part 10a. An opening 12 is formed in the side face part 10b. The opening 12 is for inserting the urine collection tool 30 that is used for collecting urine above the grains 20. In the present embodiment, a plurality of the openings 12 are formed in the side face part 10b. Specifically, two openings 12 are formed. One opening 12 is formed in the part that is connected to a short side of the bottom face part 10a, out of the four parts of the side face part 10b. The other opening 12 is formed in the part that is connected to another short side of the bottom face part 10a, out of the four parts of the side face part 10b.

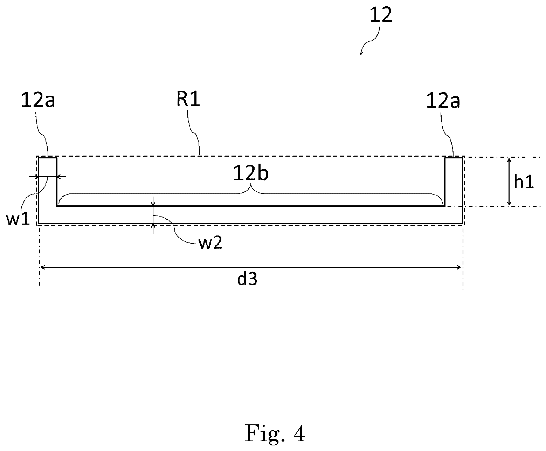

The opening 12 is apart from both the bottom face part 10a and the upper end of the side face part 10b. The distance d1 *(see* FIG. 1) from the lower end of the opening 12 to the upper end of the grains 20 is smaller than the distance d2 from the lower end of the opening 12 to the upper end of the side face part 10b. It is preferable that the distance d1 is small as much as possible, and specifically 2 cm or less. The opening 12 has a slit shape. The opening 12 has a horizontally long shape. As used herein, the horizontally long shape refers to, when considering the minimum rectangle R1 that can include the opening 12 in a front view as shown in FIG. 4, a shape in which the length of the long side of the rectangle R1 is twice or more of the length of the short side of the rectangle R1. However, the long side and the short side of the rectangle R1 are parallel, respectively, with the left/right direction (horizontal direction in FIG. 3 and FIG. 4) and the top/bottom direction (vertical direction in FIG. 3 and FIG. 4) of the opening 12. With respect to the left/right direction of the opening 12, the length d3 of the opening 12 is preferably half or more, and more preferably four-fifths or more of the inner size d4 (*see* FIG. 2) of the body portion 10. The length d3 is, for example, between 10 cm and 30 cm inclusive. Note that the length d3 is equal to the length of the long side of the rectangle R1.

The upper ends of left and right end parts 12a of the opening 12 are located higher than the upper end of a middle part 12b of the opening 12 in a front view. The middle part 12b is the part between the left and right end parts 12a. The height difference h1 between the upper ends of the left and right end parts 12a and the upper end of the middle part 12b is, for example, between 1 cm and 5 cm inclusive. The opening 12 extends horizontally in the middle part 12b, and extends upward in the left and right end parts 12a. In the present embodiment, the opening 12 extends vertically upward in the left and right end parts 12a, but the opening 12 may extend obliquely upward in the left and right end parts 12a. The widths w1, w2 of the opening 12 are preferably between 5 mm and 10 mm inclusive. The width w1 and the width w2 are the widths of the opening 12, respectively, in the left and right end parts 12a and the middle part 12b. The width w1 and the width w2 may be equal to each other, or may be different from each other. Naturally, the opening 12 does not allow an animal using the animal toilet 1 to pass therethrough.

The grains 20 are laid on the bottom face part 10a of the body portion 10. When the animal toilet 1 is used, an animal excretes in a state of getting directly on the grains 20. The grains 20 have a water absorbing property, and treat urine by absorbing it. That is, the grains 20 take urine in the inside thereof and retain it. The grains 20 having the water absorbing property require a liquid passing rate of less than 60 % measured by the following test. First, approximate 50 grams of the grains 20 (sample) are placed in a sieve with the inner diameter of 10 cm and the mesh size of 1 mm. An empty beaker is set under the sieve. Then, 30 ml of water is dripped on the sample over 10 seconds using a syringe with the inner diameter of its outer cylinder of 3 cm and the inner diameter of its cylinder tip of 4 mm (60 ml syringe manufactured by Terumo Corp.). After waiting 1 minute, the quantity of the water in the beaker is measured. The ratio of the measured water quantity with respect to the quantity of the dripped water (30 ml) shall be the liquid passing rate. That is, if the water quantity in the beaker is less than 18 ml, the liquid passing rate is less than 60 %, and therefore the grains 20 are found to have the water absorbing property.

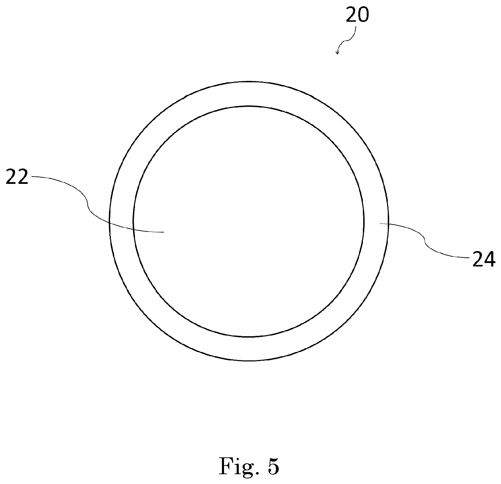

FIG. 5 is a schematic view showing the grain 20. The grain 20 has a granular shape. Examples of the granular shape include a sphere, column, and ellipsoid. The particle diameter of the grain 20 is, for example, between 5 mm and 20 mm inclusive. As used herein, the particle diameter of the grain 20 is defined as the diameter of the minimum sphere that can include the grain 20. The grain 20 contains an organic substance as its main material. As used herein, the main material of the grain 20 refers to the material that accounts for the highest weight ratio in the grain 20, out of one or more materials constituting the grain 20. The grain 20 may be made only of an organic substance, or may be made of an organic substance and an inorganic substance. Examples of the organic substance include papers, plants, plastics, and organic sludge.

The papers refer to a material made mainly of pulp. As the papers, in addition to ordinary paper (paper powder), for example, fluff pulp, paper derived from vinyl chloride wallpaper (paper generated during manufacturing or classifying vinyl chloride wallpaper), paper derived from a gypsum board (paper generated during manufacturing or classifying a gypsum board), or paper derived from a sanitary article (paper generated during manufacturing or classifying a sanitary article that contains paper) can be used. Examples of the sanitary article containing paper include paper diapers, sanitary napkins, urine absorbing pads, and sanitary paper (tissue paper, toilet paper, paper towels, or the like). As the plants, for example, wood powder, sawdust, or a plant residue (used tea leaves, bean curd lees, or the like) can be used. As the plastics, in addition to ordinary plastic, for example, plastic derived from vinyl chloride wallpaper (plastic generated during manufacturing or classifying vinyl chloride wallpaper), or plastic derived from a sanitary article (plastic generated during manufacturing or classifying a sanitary article that contains plastic) can be used. Examples of the sanitary article containing plastic include paper diapers, sanitary napkins, urine absorbing pads, and sanitary masks. As the organic sludge, for example, papermaking sludge, or pulp sludge can be used.

The grain 20 includes a core portion 22, and a coating portion 24. The core portion 22 is a granule formed in a granular shape. The core portion 22 has a function of absorbing and retaining urine. The core portion 22 contains an organic substance as its main material. As used herein, the main material of the core portion 22 refers to the material that accounts for the highest weight ratio in the core portion 22, out of one or more materials constituting the core portion 22 (core portion material).

The coating portion 24 covers the core portion 22. The coating portion 24 may cover the entire surface of the core portion 22, or may cover only a part of the surface of the core portion 22. The coating portion 24 has a function of bonding the grains 20 that have absorbed urine to agglomerate them when in use. The coating portion 24 also contains an organic substance as its main material. As used herein, the main material of the coating portion 24 refers to the material that accounts for the highest weight ratio in the coating portion 24, out of one or more materials constituting the coating portion 24 (coating material). The coating portion 24 contains an adhesive material. Examples of the adhesive material include a water-absorbent polymer, starch, CMC (carboxymethyl cellulose), PVA (polyvinyl alcohol), and dextrin. As the water-absorbent polymer, for example, an acrylic water-absorbent polymer such as sodium polyacrylate can be used.

The grains 20 can be formed as follows, for example. First, a plurality of granules that will serve as the core portions 22 are formed by granulating the core portion material with a granulation apparatus. As the granulation apparatus, for example, an extrusion granulator can be used. Prior to the granulation, pretreatment such as pulverization, kneading, and adding water is performed on the core portion material as needed. Next, the coating portion 24 is formed by attaching a powdery coating material to the surface of each core portion 22 with a coating apparatus or the like. The coating material can be attached by, for example, sprinkling or spraying. After that, posttreatment such as sieving (sizing), and drying is performed as needed. Accordingly, the plurality of grains 20 are formed.

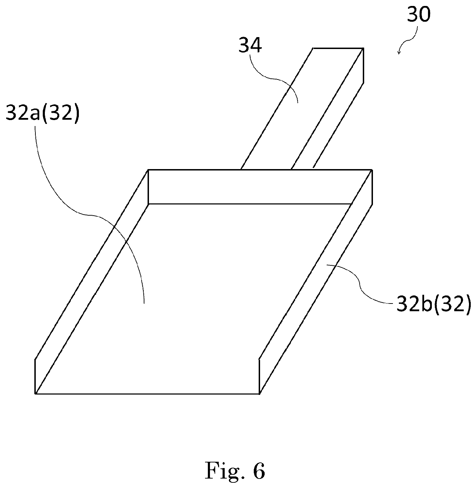

FIG. 6 is a perspective view showing the urine collection tool 30. The urine collection tool 30 is provided separately from the body portion 10. The urine collection tool 30 includes a urine accumulation portion 32, and a grip portion 34. The urine accumulation portion 32 is a portion in which urine accumulates. The urine accumulation portion 32 is composed of a plate-shaped member, and has a bottom face part 32a and a side face part 32b. The bottom face part 32a has an approximately rectangular shape in a plane view. The side face part 32b is composed of three parts that are connected, respectively, to three sides, out of the four sides of the bottom face part 32a. That is, the three sides of the bottom face part 32a are provided with the side face part 32b, whereas the remaining one side is not provided with the side face part 32b. The side that is not provided with the side face part 32b is the side located on the tip side of the bottom face part 32a (the side opposite to the grip portion 34). The thickness of the urine accumulation portion 32 is smaller than the widths w1, w2 of the opening 12. The thickness of the urine accumulation portion 32 is, for example, between 2 mm and 5 mm inclusive. The grip portion 34 is a portion designed to be held by a user's hand. The grip portion 34 has a rod shape so as to be easily grasped.

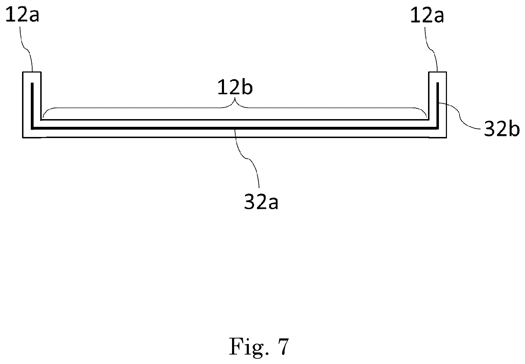

The urine collection tool 30 is capable of being inserted in and extracted from the body portion 10 through the opening 12. At this time, the bottom face part 32a passes through the middle part 12b of the opening 12, and the side face part 32b passes through the left and right end parts 12a of the opening 12 as shown in FIG. 7. The urine collection tool 30 is capable of being inserted in and extracted from the body portion 10 through the opening 12 without touching the side face part 10b (inner surface of the opening 12). The opening 12 allows the urine accumulation portion 32 to pass therethrough, but does not allow the grip portion 34 to pass therethrough. When the urine collection tool 30 is inserted in the body portion 10, the urine accumulation portion 32 is located above the grains 20 *(see* FIG. 1). As a material of the urine collection tool 30, for example, plastic or metal can be used.

Next, a method for collecting animal urine in the animal toilet 1 will be described. First, when an animal urinates getting on the grains 20, the urine collection tool 30 is inserted between the animal's rear and the grains 20 through the opening 12. The timing for inserting the urine collection tool 30 may be immediately before the urination, or may be during the urination. At this time, it is preferable that the urine collection tool 30 is slightly tilted so that the tip side thereof is positioned higher in order to prevent urine received in the urine accumulation portion 32 from spilling. When the amount of urine required for a test has accumulated in the urine accumulation portion 32, the urine collection tool 30 is pulled out from the body portion 10. The timing of pulling out the urine collection tool 30 may be after the urination, or may be during the urination. Thus, fresh urine of the animal can be collected. Urine that has not been collected by the urine collection tool 30 is absorbed by the grains 20.

The effects of the animal toilet 1 will be described. In the animal toilet 1, the side face part 10b of the body portion 10 is provided with the opening 12 for inserting the urine collection tool 30 above the grain 20. For this reason, even in the case where the space between the animal's body and the side face part 10b is small, the urine collection tool 30 can be easily inserted under the animal's rear (between the rear and the grain 20) through the opening 12 of the side face part 10b. Accordingly, the animal toilet 1 in which fresh urine can be easily collected is implemented.

Incidentally, one possible way to make it easier to insert the urine collection tool 30 under the animal's rear is to lower the side face part 10b (shorten the distance from the upper end of the grains 20 to the upper end of the side face part 10b). However, in that case, the grains 20 and animal urine become more likely to surmount the side face part 10b and spill from the body portion 10. In this regard, providing the opening 12 can make it easier to insert the urine collection tool 30 under the animal's rear without lowering the side face part 10b in the animal toilet 1.

The opening 12 has a slit shape. The opening 12 of this shape allows the urine accumulation portion 32 composed of a plate-shaped member to pass therethrough, but makes it difficult for the grains 20 to pass therethrough. For this reason, it is advantageous for preventing the grains 20 from spilling from the body portion 10 through the opening 12.

The larger the widths w1, w2 of the opening 12 are, the easier it is to insert and extract the urine collection tool 30. From this viewpoint, the widths w1, w2 are preferably 5 mm or more. On the other hand, if the widths w1, w2 are too large, the grains 20 are likely to spill from the body portion 10 through the opening 12. From this viewpoint, the widths w1, w2 are preferably 10 mm or less.

The opening 12 has a horizontally long shape. The opening 12 of this shape is suitable for allowing the urine collection tool 30 (urine accumulation portion 32) having a horizontally long shape to pass therethrough. There is an advantage that the urine collection tool 30 having a horizontally long shape is easily inserted between the animal's rear and the grains 20.

Enlarging the length d3 of the opening 12 is advantageous for receiving animal urine over a wide range. From this viewpoint, the length d3 is preferably half or more, and more preferably four-fifths or more of the inner size d4 of the body portion 10.

The upper ends of left and right end parts 12a of the opening 12 are located higher than the upper end of the middle part 12b of the opening 12. Thus, the urine collection tool 30 (urine accumulation portion 32) having a similar shape with the opening 12 can be inserted in the body portion 10 through the opening 12. The urine collection tool 30 of this shape has an advantage that urine is unlikely to spill over left and right ends thereof.

The opening 12 extends horizontally in the middle part 12b, and extends upward in the left and right end parts 12a. Thus, the urine collection tool 30 (urine accumulation portion 32) having a similar shape with the opening 12 can be inserted in the body portion 10 through the opening 12. The urine collection tool 30 having this shape has an advantage that urine can be widely and shallowly stored in a horizontal part (the bottom face part 32a). Storing urine shallowly in this way can make urine less likely to touch the side face part 10b (inner surface of the opening 12) when the urine collection tool 30 is extracted from the body portion 10.

The plurality of openings 12 are formed in the side face part 10b. In this case, it is possible to select the opening 12 closest to the rear of an animal that is urinating. Thus, the urine collection tool 30 can be inserted under the animal's rear more surely. However, it is not essential that the plurality of openings 12 are formed in the side face part 10b. Only one opening 12 may be formed in the side face part 10b.

The urine collection tool 30 includes the urine accumulation portion 32, and the grip portion 34. By providing the grip portion 34 in addition to the urine accumulation portion 32 in this way, the urine collection tool 30 can be easily inserted in and extracted from the body portion 10.

The opening 12 allows the urine accumulation portion 32 to pass therethrough, but does not allow the grip portion 34 to pass therethrough. Thus, it is possible to avoid a situation in which even the grip portion 34 enters inside the body portion 10, when the urine collection tool 30 is inserted in the body portion 10.

The urine collection tool 30 is capable of being inserted in and extracted from the body portion 10 through the opening 12 without touching the side face part 10b. In this case, because there is a margin in both the left/right direction and the top/bottom direction of the opening 12, the urine collection tool 30 can be smoothly inserted in and extracted from the body portion 10.

The animal toilet 1 is provided with the urine collection tool 30. For this reason, users can collect animal urine using the urine collection tool 30 without preparing the urine collection tool 30 separately from the animal toilet 1. However, it is not essential that the animal toilet 1 is provided with the urine collection tool 30.

The grains 20 having a water absorbing property are laid on the bottom face part 10a of the body portion 10. In this case, since urine is absorbed by the grains 20, it is difficult to collect urine that has touched the grains 20. Therefore, the animal toilet 1 in which it is possible to collect urine that has not touched the grains 20 is particularly useful.

The grain 20 includes the coating portion 24 containing an adhesive material, in addition to the core portion 22. Thus, it becomes easy to remove only the grains 20 after use from the body portion 10, because an agglomeration composed of a plurality of grains 20 that have absorbed urine can be obtained. However, it is not essential that the grains 20 have multi-layer structure (double-layer structure) composed of the core portion 22 and the coating portion 24. The grains 20 may have single-layer structure composed only of the core portion 22.

The grains 20 contain an organic substance as a main material. In this case, it is possible to obtain the grains 20 suitable for being disposed of by incineration. This contributes to convenience of disposal of the grains 20 after use. Particularly in the case where grains 20 are made only of an organic substance, it is possible to obtain the grains 20 more suitable for being disposed of by incineration. However, it is not essential that the grains 20 contain an organic substance as a main material. The grains 20 may contain an inorganic substance as a main material.

The body portion 10 is composed of one container. In this case, it is possible to implement the animal toilet 1 with simple constitution, compared to a case where the body portion 10 is composed of a plurality of containers.

Incidentally, cats have the habit of hiding their excrement with grains after excretion, unlike other animals such as dogs. Therefore, the animal toilet 1 in which the grains 20 are laid in the body portion 10 is particularly suitable for being used as a cat toilet.

### (Second Embodiment)

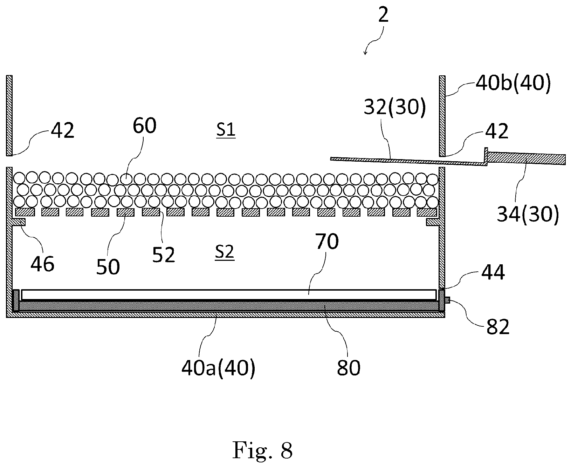

FIG. 8 is an end view showing a second embodiment of an animal toilet according to the present invention. An animal toilet 2 includes a body portion 40, a partition portion 50, a grain 60, a water-absorbing sheet 70, a drawer portion 80, and the urine collection tool 30. The constitution of the urine collection tool 30 is as described in the first embodiment. The animal toilet 2 is, for example, a cat toilet. The body portion 40 is composed of one box-shaped container, and has a bottom face part 40a and a side face part 40b. The body portion 40 receives excreted urine. A plurality of the grains 60 for treating animal urine are laid in the body portion 40. The outer shape of the body portion 40 is an approximately rectangular parallelepiped shape. As a material of the body portion 40, for example, plastic such as polypropylene or polyethylene can be used.

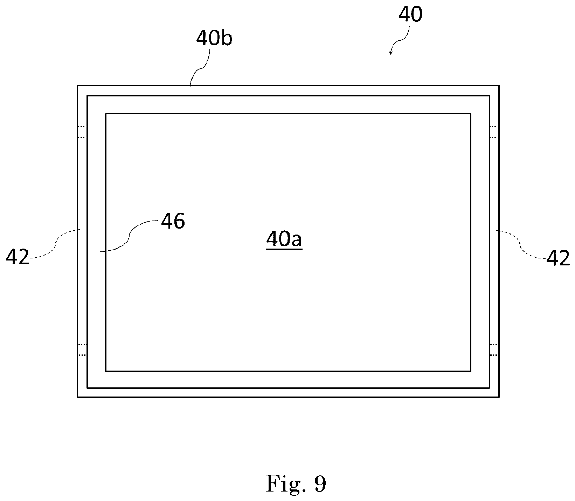

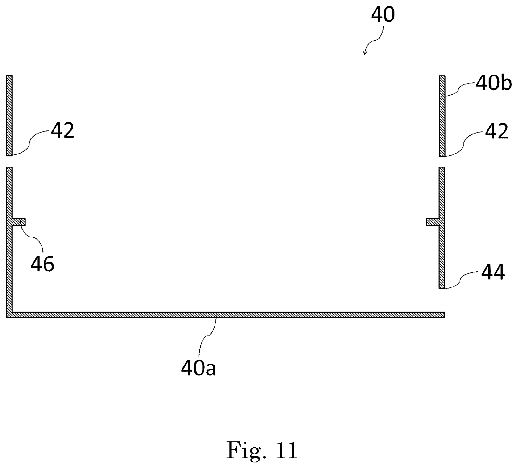

FIG. 9, FIG. 10 and FIG. 11 are, respectively, a plane view, a front view and an end view showing the body portion 40. The bottom face part 40a has an approximately rectangular shape in a plane view. The side face part 40b is composed of four parts that are connected, respectively, to the four sides of the bottom face part 40a. An opening 42 is formed in the side face part 40b. The opening 42 is for inserting the urine collection tool 30 above the grains 60. In the present embodiment, a plurality of the openings 42 are formed in the side face part 40b. Specifically, two openings 42 are formed. One opening 42 is formed in the part that is connected to one short side of the bottom face part 40a, out of the four parts of the side face part 40b. The other opening 42 is formed in the part that is connected to another short side of the bottom face part 40a, out of the four parts of the side face part 40b. The constitution of the opening 42 is the same as the constitution of the opening 12.

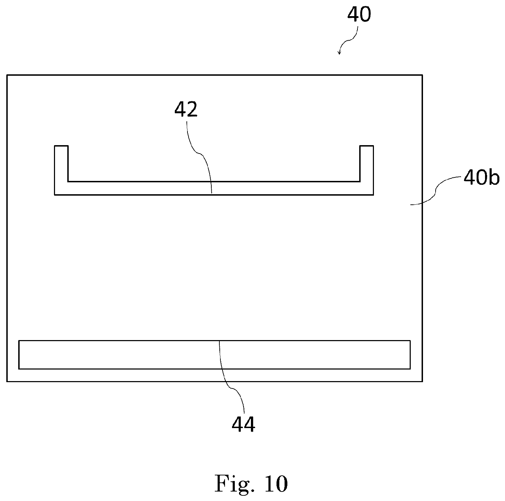

An opening 44 for inserting and extracting the drawer portion 80 is also formed in the side face part 40b. Only one opening 44 is formed in the side face part 40b. The opening 44 is formed in the part that is connected to the one short side of the bottom face part 40a, out of the four parts of the side face part 40b. Therefore, both the opening 42 and the opening 44 are formed in that part of the side face part 40b. However, the opening 42 is located higher than the opening 44. The opening 44 is located near the bottom face part 40a, and in a horizontally long rectangular shape. With respect to the left/right direction (horizontal direction in FIG. 10) of the opening 44, the length of the opening 44 is nearly equal to the inner size of the body portion 40, and is, for example, between 20 cm and 40 cm inclusive. With respect to the top/bottom direction (vertical direction in FIG. 10) of the opening 44, the length of the opening 44 is, for example, between 2 cm and 5 cm inclusive.

The body portion 40 has a support part 46. The support part 46 projects from the side face part 40b toward the inside of the body portion 40. The support part 46 supports the partition portion 50 from below. The support part 46 is provided over the entirety of the inner periphery of the side face part 40b in a plane view (*see* FIG. 9). That is, the support part 46 is composed of a projected rim that is provided annularly along the inner periphery of the side face part 40b. The projecting length of the support part 46 (the length in the direction perpendicular to the inner surface of the side face part 40b provided with the support part 46) is, for example, between 5 mm and 15 mm inclusive. The support part 46 may be formed integrally with the side face part 40b, or may be attached to the side face part 40b after being formed separately from the side face part 40b.

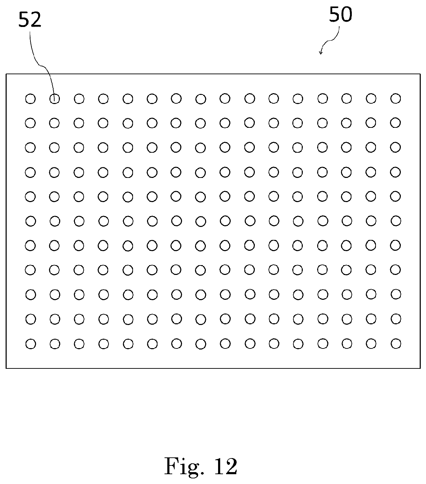

FIG. 12 is a plane view showing the partition portion 50. The partition portion 50 is placed on the support part 46. The partition portion 50 is not fixed to the body portion 40. The partition portion 50 is detachable relative to the body portion 40. The partition portion 50 is capable of being disposed in the body portion 40 in a state in which the entire periphery of the partition portion 50 is apart from the side face part 40b. The partition portion 50 is composed of a plate-shaped member. The partition portion 50 has an approximately rectangular shape in a plane view. The partition portion 50 is disposed parallel with the bottom face part 40a. Moreover, the partition portion 50 is disposed at a position apart from both the upper end of the body portion 40 (side face part 40b) and the water-absorbing sheet 70. Thus, the partition portion 50 divides the internal space of the body portion 40 into an upper space S1 and a lower space S2. The upper space S1 is present above the partition portion 50, and is a space in which the grains 60 are laid. The lower space S2 is present below the partition portion 50, and is a space in which urine that has not been collected by the urine collection tool 30 accumulates. As a material of the partition portion 50, for example, plastic such as polypropylene or polyethylene can be used.

The partition portion 50 has a through hole 52 that allows urine to pass therethrough. The through hole 52 allows urine to pass therethrough, but does not allow the grain 60 to pass therethrough. The partition portion 50 has a plurality of the through holes 52. The plurality of through holes 52 are arranged two-dimensionally in the partition portion 50. The plane shape of each through hole 52 is a circle. The diameter of each through hole 52 is, for example, between 2 mm and 4 mm inclusive.

The grains 60 are laid in the upper space S1 of the body portion 40. When the animal toilet 2 is used, an animal excretes in a state of getting directly on the grains 60. The grains 60 have a hydrophobic property, and treat urine by having the urine pass while winding its way through gaps between the plurality of grains 60. That is, the grains 60 have the property of not absorbing liquid such as urine at all, or hardly absorbing it. The grains 60 having the hydrophobic property require the liquid passing rate of 60 % or more measured by the test described above.

The grain 60 has a granular shape. The particle diameter of the grain 60 is, for example, between 5 mm and 20 mm inclusive. The grain 60 contains an organic substance as a main material. The grain 60 may be made only of an organic substance, or may be made of an organic substance and an inorganic substance. Examples of the organic substance include papers, plants, plastics, and organic sludge. As the papers, in addition to those described above, for example, photographic paper, or release paper may be used. As the plastics, in addition to those described above, for example, an aluminum deposited film may be used. In a case where a material not having a hydrophobic property is used as a material of the grains 60, the material may be subjected to hydrophobic treatment (water repellency treatment) in advance.

The material(s) constituting the grain 60 may be only one material, or two or more materials. In the former case, the main material described above is the only material constituting the grain 60. In the latter case, the grain 60 is made of a mixture of the main material and other material(s). Examples of the other material include gypsum and baking soda. Adding gypsum or baking soda makes it easier to give a hydrophobic property to the grain 60. The quantity of gypsum or baking soda is, for example, 5 wt.% or more and less than 50 wt.% with respect to the entirety of the grain 60. The grain 60 has single-layer structure composed only of an uncovered granule.

The grains 60 can be manufactured by, for example, the following method. First, granules that will serve as the grains 60 are formed by granulating a granulating material (material(s) constituting the grain 60) with a granulation apparatus. As the granulation apparatus, for example, an extrusion granulator can be used. The granules may be subjected to hydrophobic treatment as needed. The hydrophobic treatment can be performed by, for example, coating the surfaces of the granules with a hydrophobic agent (water repellent agent). In the case where the hydrophobic treatment is not performed, it is preferable that crevices are prevented from forming in the granules as much as possible by increasing the pressure that is applied to the granulating material during granulation. This is because the crevices serve as a path through which liquid such as urine enters inside the grains 60. Prior to the granulation, pretreatment such as pulverization, kneading, and adding water is performed on the granulating material as needed. Also, after the granulation, posttreatment such as sieving (sizing), and drying is performed as needed. Accordingly, the plurality of grains 60 are formed.

The water-absorbing sheet 70 is disposed in the lower space S2. The water-absorbing sheet 70 absorbs urine that has passed through the through hole 52 of the partition portion 50. That is, urine that has passed through the through hole 52 accumulates in the lower space S2 in the state of being absorbed by the water-absorbing sheet 70.

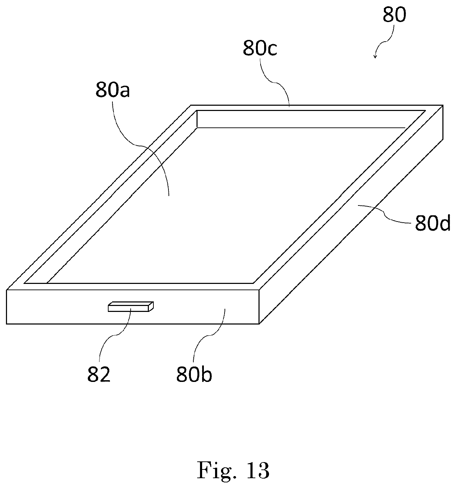

FIG. 13 is a perspective view showing the drawer portion 80. The drawer portion 80 has a bottom board 80a, a front board 80b, a rear board 80c, and a pair of side boards 80d. The size of the bottom board 80a is nearly equal to the size of the bottom face part 40a of the body portion 40. The front board 80b has nearly the same shape and size as the opening 44. A grip 82 is attached to the front board 80b. The drawer portion 80 is capable of being inserted in and extracted from the body portion 40 through the opening 44 formed in the side face part 40b. The drawer portion 80 houses the water-absorbing sheet 70. That is, the water-absorbing sheet 70 is disposed in the body portion 40 in the state of being housed in the drawer portion 80. As a material of the drawer portion 80, for example, plastic such as polypropylene or polyethylene can be used.

Next, a method for collecting animal urine in the animal toilet 2 will be described. First, when an animal urinates getting on the grains 60, the urine collection tool 30 is inserted between the animal's rear and the grains 60 through the opening 42. When the amount of urine required for a test has accumulated in the urine accumulation portion 32, the urine collection tool 30 is pulled out from the body portion 40. Thus, fresh urine of the animal can be collected. Urine that has not been collected by the urine collection tool 30 moves from the upper space S1 to the lower space S2 via the through holes 52 of the partition portion 50 after passing through gaps between the grains 60. The urine that has moved to the lower space S2 is absorbed by the water-absorbing sheet 70.

The effects of the animal toilet 2 will be described. In the animal toilet 2, the side face part 40b of the body portion 40 is provided with the opening 42 for inserting the urine collection tool 30 above the grain 60. For this reason, even in the case where the space between the animal's body and the side face part 40b is small, the urine collection tool 30 can be easily inserted under the animal's rear (between the rear and the grain 60) through the opening 42 of the side face part 40b. Accordingly, the animal toilet 2 in which fresh urine can be easily collected is implemented.

The partition portion 50 is provided that has the through holes 52 allowing animal urine to pass therethrough, and divides the internal space of the body portion 40 into the upper space S1 and the lower space S2. Thus, it is possible to make a malodor of urine less likely to leak outside the animal toilet 2, because the urine can be stored in the lower space S2.

The grains 60 having a hydrophobic property are laid in the upper space S1 of the body portion 40. In this case, it is also possible to collect urine that has passed through the grains 60 and test it, but the components of the grains 60 may affect the test results. Therefore, also in this case, the animal toilet 2 in which it is possible to collect urine that has not touched the grains 60 is useful.

The partition portion 50 is not fixed to the body portion 40. In this case, the partition portion 50 can be attached to and detached from the body portion 40 easily.

The partition portion 50 is placed on the support part 46. Thus, the partition portion 50 can stay at a predetermined position in the body portion 40 without being fixed to the body portion 40.

The support part 46 is provided over the entirety of the inner periphery of the side face part 40b in a plane view. Thus, it is possible to make a situation less likely to occur in which the partition portion 50 falls into the lower space S2.

The partition portion 50 is capable of being disposed in the body portion 40 in the state in which the entire periphery thereof is apart from the side face part 40b. By providing a margin between the partition portion 50 and the side face part 40b in this way, the partition portion 50 can be smoothly attached to and detached from the body portion 40.

The water-absorbing sheet 70 is disposed in the lower space S2. Thus, urine accumulating in the lower space S2 can be confined in the water-absorbing sheet 70. For this reason, it is possible to make a malodor of urine even less likely to leak outside the animal toilet 2.

The drawer portion 80 is provided that is capable of being inserted in and extracted from the body portion 40. Thus, it is possible to easily carry out the work of replacing a used water-absorbing sheet 70 with a new one. Other effects of the animal toilet 2 are the same as the animal toilet 1.

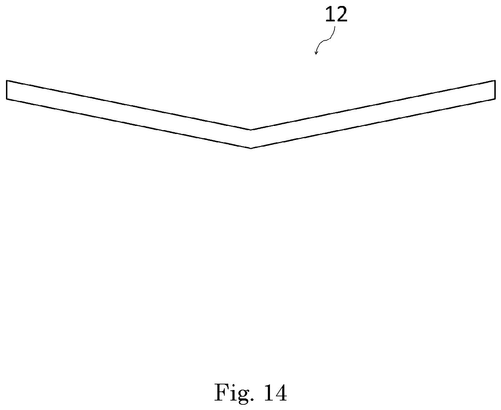

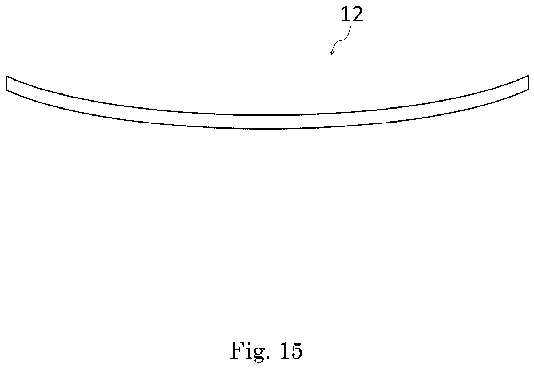

The present invention is not limited to the above-described embodiments, and various modifications can be made. In the above-described embodiments, an example is given in which the opening 12 extends horizontally in the middle part 12b, and extends upward in the left and right end parts 12a. However, the opening 12 may have such a shape that the position of the upper end becomes higher gradually from the center toward the both ends of the opening 12 with respect to the left/right direction of the opening 12, as shown, for example, in FIG. 14 and FIG. 15. In FIG. 14, the opening 12 has a V-shape in a front view. Also, in FIG. 15, the opening 12 has a convex downward curved shape in a front view. In these cases, a urine collection tool 30 corresponding to the shape of the opening 12 is used. That is, a urine collection tool 30 is used in which the cross section of the urine accumulation portion 32 (cross section perpendicular to the insertion direction of the urine collection tool 30) has a V-shape or convex downward curved shape. Similarly, the opening 42 may have such a shape that the position of the upper end becomes higher gradually from the center toward the both ends of the opening 42 with respect to the left/right direction of the opening 42.

### List of Reference Numerals

- 1: Animal Toilet
- 10: Body Portion
- 10a: Bottom Face Part
- 10b: Side Face Part
- 12: Opening
- 12a: Left and Right End Parts
- 12b: Middle Part
- 20: Grain
- 22: Core Portion
- 24: Coating Portion
- 30: Urine Collection Tool
- 32: Urine Accumulation Portion
- 32a: Bottom Face Part
- 32b: Side Face Part
- 34: Grip Portion
- 40: Body Portion
- 40a: Bottom Face Part
- 40b: Side Face Part
- 42: Opening
- 44: Opening
- 46: Support Part
- 50: Partition Portion
- 52: Through Hole
- 60: Grain
- 70: Water-Absorbing Sheet
- 80: Drawer Portion
- 82: Grip

## Claims

1. An animal toilet comprising:
a body portion in which a grain for treating urine of an animal is laid, the body portion having a bottom face part and a side face part,
wherein the side face part is provided with an opening for inserting a urine collection tool used for collecting the urine above the grain.

2. The animal toilet according to claim 1,
wherein the opening has a slit shape.

3. The animal toilet according to claim 2,
wherein a width of the opening is between 5 mm and 10 mm inclusive.

4. The animal toilet according to claim 1 or 2,
wherein the opening has a horizontally long shape.

5. The animal toilet according to claim 4,
wherein with respect to left/right direction of the opening, a length of the opening is half or more of an inner size of the body portion.

6. The animal toilet according to claim 5,
wherein the length of the opening is four-fifths or more of the inner size of the body portion.

7. The animal toilet according to claim 4,
wherein upper ends of left and right end parts of the opening are located higher than an upper end of a middle part of the opening in a front view.

8. The animal toilet according to claim 7,
wherein the opening extends horizontally in the middle part, and extends upward in the left and right end parts.

9. The animal toilet according to claim 1 or 2,
wherein the side face part is provided with a plurality of the openings.

10. The animal toilet according to claim 1 or 2, further comprising:
the urine collection tool.

11. The animal toilet according to claim 10,
wherein the urine collection tool includes a urine accumulation portion in which the urine accumulates, and a grip portion designed to be held by hand.

12. The animal toilet according to claim 11,
wherein the opening allows the urine accumulation portion to pass therethrough, but does not allow the grip portion to pass therethrough.

13. The animal toilet according to claim 10,
wherein the urine collection tool is capable of being inserted in and extracted from the body portion through the opening without touching the side face part.

14. The animal toilet according to claim 1 or 2, further comprising:
the grain that is laid on the bottom face part, and has a water absorbing property.

15. The animal toilet according to claim 14,
wherein the grain includes a core portion formed in a granular shape, and a coating portion that contains an adhesive material and covers the core portion.

16. The animal toilet according to claim 14,
wherein the grain contains an organic substance as a main material.

17. The animal toilet according to claim 1 or 2, further comprising:
a partition portion that has a through hole allowing the urine to pass therethrough, and divides an internal space of the body portion into an upper space and a lower space.

18. The animal toilet according to claim 17, further comprising:
the grain that is laid in the upper space, and has a hydrophobic property.

19. The animal toilet according to claim 17,
wherein the partition portion is not fixed to the body portion.

20. The animal toilet according to claim 19,
wherein the body portion has a support part that projects from the side face part toward an inside of the body portion, and
the partition portion is placed on the support part.

21. The animal toilet according to claim 20,
wherein the support part is provided over an entire inner periphery of the side face part in a plane view.

22. The animal toilet according to claim 17,
wherein the partition portion is capable of being disposed in the body portion in a state in which an entire periphery of the partition portion is apart from the side face part.

23. The animal toilet according to claim 1 or 2,
wherein the body portion is composed of one container.

24. The animal toilet according to claim 17, further comprising:
a water-absorbing sheet that is disposed in the lower space, and absorbs the urine.

25. The animal toilet according to claim 1 or 2,
wherein the animal toilet is a cat toilet.
